# EUROPEAN PATENT SPECIFICATION

(11) **EP 1 565 111 B1**
(45) Date of publication and mention of the grant of the patent: **23.03.2011**
(21) Application number: 03808155.0
(22) Date of filing: 06.10.2003
(51) Int. Cl.: A61B 17/02

(54) **ENDOSCOPIC RETRACTOR**
ENDOSKOPISCHER RETRAKTOR
ECARTEUR ENDOSCOPIQUE

(30) Priority: 04.10.2002 US 416370 P
(43) Date of publication of application: 24.08.2005
(60) Divisional application: 10190991.9
(73) Proprietor: Tyco Healthcare Group LP, New Haven, CT 06511 (US)
(72) Inventor: STEARNS, Ralph, A., Bozrah, CT 06334 (US); ORBAN, Joseph, P., III, Norwalk, CT 06850 (US); VIOLA, Frank, J., Sandy Hook, CT 06482 (US)
(74) Representative: Soames, Candida Jane
(86) International application number: PCT/US2003/031650
(87) International publication number: WO 2004/032757

(56) References cited:
- WO-A-93/13713
- US-A- 6 067 990
- US-B1- 6 248 062

## Description

### CROSS-REFERENCE TO RELATED APPLICATIONS

The present application claims the benefit of and priority to U.S. Provisional Application Serial No. 60/416,370, filed on October 4, 2002.

### BACKGROUND

### 1. Technical Field

The present disclosure relates to endoscopic surgical retractors and organ manipulators for use during minimally invasive surgical procedures and, more particularly, to endoscopic surgical retractors which are configurable from a first position for insertion through a trocar or surgical sheath to a second position to facilitate retraction and/or manipulation of organs or tissue.

### 2. Background of Related Art

It is well established that the performance of various types of surgical procedures using minimally invasive techniques and instrumentation has provided numerous physical benefits to the patient while reducing the overall cost of such procedures. Endoscopic surgical procedures have been used for many years and the popularity of such procedures continues to increase. For example, more and more surgeons are complementing traditional open methods of gaining access to vital organs and body cavities with endoscopes and endoscopic instruments which access organs through small puncture-like incisions.

Once inserted into the initial incision, a trocar provides a narrow passageway for endoscopic instruments which are inserted into the patient through a cannula or port disposed within the trocar. As can be appreciated, access to the surgical cavity is typically limited to the internal dimensions of the trocar channel and the size of the cannula. It is believed that minimizing the size of the incision minimizes pain and provides other benefits to the patient. Smaller cannulas are usually preferred during most endoscopic procedures which, ultimately, present a design challenge to instrument manufacturers who must find ways to make surgical instruments which will fit through the small cannulas.

For example, it is known that the ability to manipulate organs and tissue within the operating cavity is important during endoscopic procedures due to the limited view within the operating cavity. Utilizing a traditional open retractor to manipulate organs and/or retract tissue is not an option since it would force a surgeon to forego the benefits of minimally invasive surgery. As a result, manufacturers have been challenged to design various types of endoscopic retractors which can fit through small cannulas and which do not limit a surgeon's ability to retract and/or manipulate organs and tissue as needed during the surgical procedure.

Several endoscopic retractors have been designed in the past but for the most part and by and large these instruments are overly complex and only allow relatively limited positioning or repositioning of organs during surgery. A need exists to develop a retractor which is simple and effective in manipulating organs to provide adequate visualization of the operating cavity for the surgeon during endoscopic surgical procedures.

A retractor according to the preamble of claim 1 is known from U.S. Patent No. 6,067,990.

### SUMMARY

The present disclosure relates to endoscopic retractors for retracting organs and the like. According to the invention, a retractor according to claim 1 is provided. It includes a shaft having at least a first section having a first mechanical interface and a second section having a second mechanical interface for engaging the first mechanical interface, the first section and the second section being selectively movable from a first, generally longitudinally-aligned configuration along an axis defined through the shaft and the first mechanical interface is disengaged from the second mechanical interface, to a second configuration wherein the second section is disposed at an angle relative to a longitudinal axis of the shaft and the first mechanical interface is engaged with the second mechanical interface. The retractor further includes at least one cable extending through the shaft and is operatively secured to the second section. The cable is remotely actuatable to move the second section from the first to the second configuration upon selective translation of the cable.

The first and second mechanical interfaces desirably cooperate to align the first section and the second section and engage the first section and second section with one another upon movement from the first configuration to the second configuration.

The first and second sections can include complementary cam-like interfaces. The cam-like interfaces rotatably and translatably engage one another upon actuation of the cable for movement from the first configuration to the second configuration. Accordingly, the first section and the second section can rotate and translate with respect to one another. The shaft can include an outer sleeve which houses the first and second sections.

At least one of the first section and the second section includes a tongue which engages a corresponding recess disposed within the other of the first section and the second section. The tongue facilitates alignment and engagement of the first section and the second section relative to one another during movement from the first configuration to the at least one additional second configuration.

The retractor includes a hinge disposed between the first section and the second section. In one embodiment, the hinge is a living hinge disposed between the first section and the second section. It is envisioned that one of the first section and the second section can include a stop for controlling the angular disposition of the first section and the second section when disposed in the at least one additional second configuration.

### BRIEF DESCRIPTION OF THE DRAWINGS

Other objects and features of the present disclosure will become apparent from the following detailed description considered in connection with the accompanied drawings. It should be understood, however, that the drawings are designed for the purpose of illustration only and not as a definition of the limits of the disclosure.

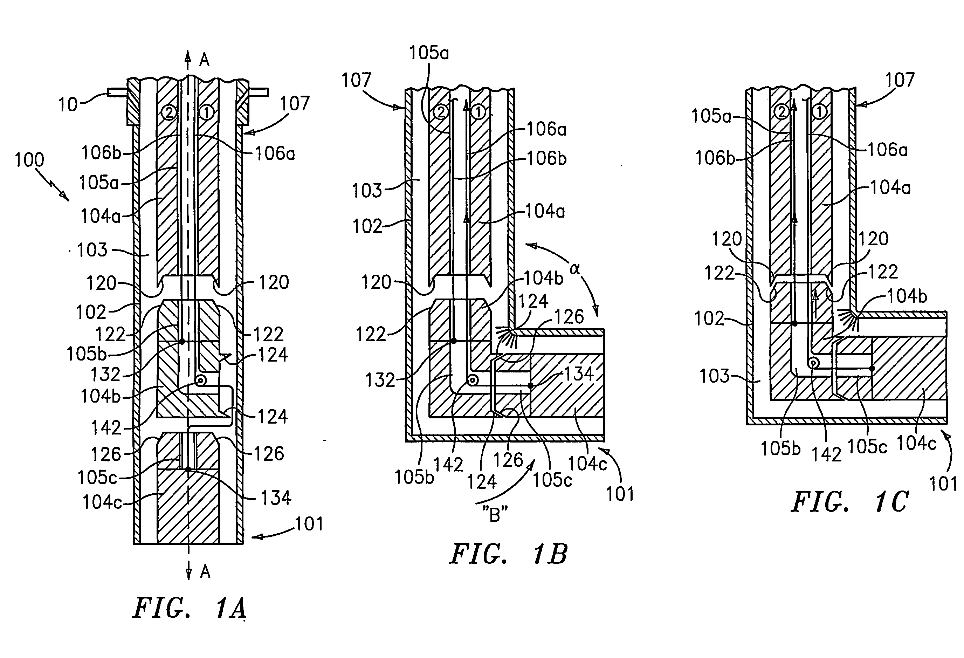

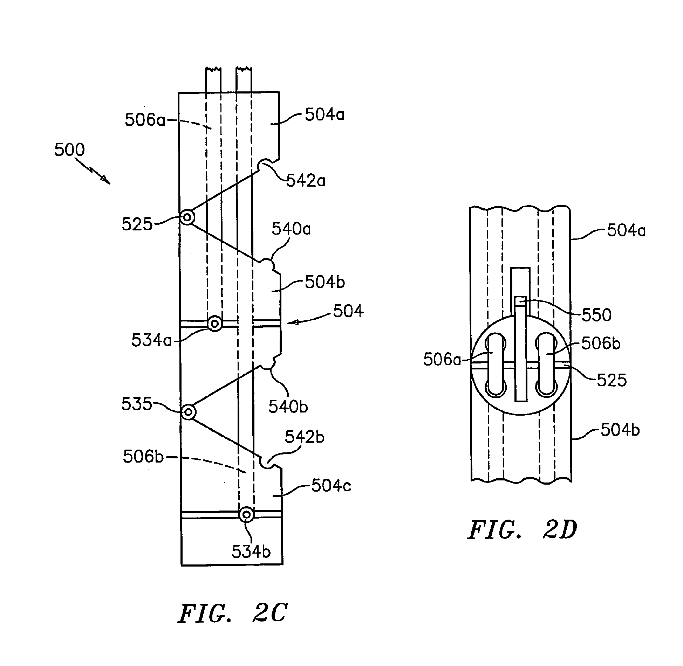

Illustrative embodiments of the subject surgical instrument are described herein with reference to the drawings wherein only the retractors shown in Figs. 2D, 4A-4D, 6A-6C, and 7A-7C are embodiments of the invention whereas the retractors shown in Figs. 1A- 2C, 3A-3B, 5A-5C, and 8A-11B do not fall under the scope of the claims:
FIG. 1A is a side sectional view of an endoscopic retractor
   shown configured for insertion through a trocar assembly;
FIG. 1B is a side sectional view of an retractor in accordance with the retractor of FIG. 1A showing a first stage of deployment wherein a third section of the retractor is rotated into position for retracting tissue;
FIG. 1C is a side sectional view of an retractor in accordance with the retractor of FIGS. 1A and 1B showing a second stage of deployment wherein a second section of the retractor is translated to engage and lock against a first section of the retractor to position the retractor for retracting tissue;
Fig. 1D is a perspective view of a retractor
wherein a chamfered tongue-like fitting is utilized to facilitate engagement of the second section and the first section to one another,
FIG. 1E is a cross-sectional view, taken along line 1E - 1E in FIG. 1D, showing a pair of cables which are used to remotely translate the second section relative to the first section;
FIG. 1F is an cross-sectional view, taken along line 1F - 1F in FIG. 1D, showing a ribbon-like cable which is used to remotely translate the second section relative to the first section;
FIG. 1G is a perspective view of a mechanical interface in a retractor in accordance with a further example, showing a first section and a second section;
FIG. 1H is a perspective view showing the mechanical interface in accordance with the retractor of FIG. 1G, showing the second section in a different position;
FIG. 1I is a perspective view of a first section in accordance with an alternate example;
FIG. 1J is a perspective view illustrating a first section and second section in accordance with the example of FIG. 1I;
FIG. 2A is a side elevational view of an endoscopic retractor in accordance with another example of the present disclosure;
FIG. 2B is a side elevational view of an endoscopic retractor in accordance with the example of FIG. 2A showing the retractor positioned for retracting tissue;
FIG. 2C is a side elevational view of an endoscopic retractor in accordance with a further example having three sections;
FIG. 2D is a front elevational view of an endoscopic retractor in accordance with an embodiment showing a locking mechanism;
FIG. 3A is a side elevational view of an endoscopic retractor in accordance with yet another example of the present disclosure;
FIG. 3B is a side elevational view of the endoscopic retractor of FIG. 3A showing the retractor positioned for retracting tissue;
FIG. 4A is a schematic side elevational view of an endoscopic organ retractor in accordance with another embodiment of the present disclosure, shown in a first or extended condition;
FIG. 4B is a schematic side elevational view of the endoscopic organ retractor in accordance with the embodiment of FIG. 4A, shown in a second or partially retracted condition;
FIG. 4C is a schematic side elevational view of the endoscopic organ retractor in accordance with the embodiment of FIGS. 4A and 4B, shown in a third or fully retracted condition;
FIG. 4D is a perspective detail view of the indicated area shown in FIG. 4A of the endoscopic organ retractor in accordance with the embodiment of FIGS. 4A - 4C;
FIG. 5A is a perspective view of a first section of an endoscopic organ retractor in accordance with still another example of the present disclosure;
FIG. 5B is a perspective view of a second section of the endoscopic organ retractor in accordance with the example of FIG. 5A;
FIG. 5C is a perspective view of the endoscopic retractor in accordance with the example of FIGS. 5A and 5B;
FIG. 6A is a front elevational view of an endoscopic retractor in accordance with a further embodiment of the present disclosure;
FIG. 6B is a side elevational view of the endoscopic retractor in accordance with the embodiment of FIG. 6A;
FIG. 6C is a front elevational view of the endoscopic retractor in accordance with another embodiment;
FIG. 7A is a left side elevational view of an endoscopic retractor in accordance with yet another embodiment of the present dislosure;
FIG. 7B is a front elevational view of the endoscopic retractor in accordance with the embodiment of FIG. 7A;
FIG. 7C is a right side elevational view of the endoscopic retractor in accordance with the embodiment of FIGS. 7A and 7B;
FIG. 8A is a front elevational view of an endoscopic retractor in accordance with still another example of the present disclosure;
FIG. 8B is a side elevational view of the endoscopic retractor in accordance with the example of FIG. 8A;
FIG. 9A is a front perspective view of an endoscopic retractor in accordance with still another example of the present disclosure, shown in a first or disassembled configuration, wherein a series of finger elements cooperate to retract tissue;
FIG. 9B is a front perspective view of the endoscopic retractor in accordance with the example of FIG. 9A shown in a second or assembled configuration;
FIGS. 10A is a front elevational view of an endoscopic retractor in accordance with still another example of the present disclosure and having a scoop-like configuration for retracting tissue, shown in a first or disassembled configuration;
FIG. 10B is a front perspective view of the endoscopic retractor in accordance with the example of FIG. 10A shown in a second or assembled configuration; and
FIGS. 11A and 11B are schematic illustrations of an endoscopic retractor in accordance with another example of the present disclosure, wherein FIG. 11A shows the organ retractor in a first or insertion/withdrawal configuration, and FIG. 11B shows the organ retractor in a second or retracted configuration.

### DETAILED DESCRIPTION OF PREFERRED EMBODIMENTS

In the drawings and in the description which follows, the term "proximal", as used in the technical field, will refer to the end of the surgical device or instrument of the present disclosure which is closest to the operator, while the term "distal" will refer to the end of the device or instrument which is furthest from the operator.

Referring now in specific detail to the drawings, in which like reference numerals identify similar or identical elements, FIGS. 1A - 1J illustrate an endoscopic organ retractor, generally designated as 100.

5 As seen in particular in FIGS. 1A - 1C, organ retractor 100 includes an elongated tube 102, preferably flexible, having a lumen 103 defining a longitudinal axis "A" extending therethrough. Lumen 103 of tube 102 is desirably configured and dimensioned to house three interacting sections, namely, a third section 104a, a second section 104b and a first section 104c. The three sections 104a, 104b and 104c 0 are desirably configured to be movable from a first configuration wherein sections 104a, 104b and 104c are generally aligned along longitudinal axis "A" to at least one additional second configuration wherein sections 104a, 104b and 104c engage one another and configure and/or shape retractor 100 in a manner to retract tissue. The first section 104c is disposed at a distal end 101 of retractor 100 and the third section 5 104a is disposed at a proximal end 107 of retractor 100.

First section 104c includes a first mechanical interface 126, formed at a proximal end 125 thereof, which is configured and dimensioned to be engagable with a corresponding and/or complementary second mechanical interface 124 desirably formed in a side surface 122 of second section 104b. Similarly, third section 104a includes a third mechanical interface 120, formed at a distal end 128 thereof, which is configured and dimensioned to be engagable with a corresponding and/or complementary fourth mechanical interface 122 desirably formed in a proximal end 130 of second section 104b.

Retractor 100 includes a first cable 106a extending through a longitudinally oriented passage 105a formed in third section 104a, into second section 104b through a proximal longitudinally oriented portion of an L-shaped passage 105b and out second section 104b through a radially oriented portion of L-shaped passage 105b, and affixed to first section 104c at an attachment point 134, preferably located in a bore 105c formed in and extending proximally out of first section 104c. Retractor 100 further includes a second cable 106b extending through passage 105a of third section 104a and affixed to second section 104b at an attachment point 132, preferably located within L-shaped passage 105b. Cables 106a and 106b are remotely translatable by the surgeon to effectuate manipulation of retractor 100 between the first and second configuration. A series of cable guides 142 may be used to facilitate translation of cables 106a and 106b through and/or into sections 104a, 104b and 104c.

As seen in FIGS. 1A - 1E, cables 106a, 106b are preferably offset a distance from longitudinal axis "A". Most preferably, at least cable 106a is offset from longitudinal axis "A".

In use and as best illustrated in FIGS. 1A - 1C, retractor 100 is initially configured as shown in FIG. 1A (e.g., sections 104a-104c substantially longitudinally aligned with one another along longitudinal axis "A") to facilitate insertion of retractor 100 through a trocar assembly 10. Once the retractor 100 is inserted a desired and/or a sufficient distance, through trocar assembly 10 into the operative site, the surgeon remotely actuates first cable 106a (e.g., pulls on first cable 106a in a proximal direction) thereby causing first section 104c to rotate in the direction of arrow "B", slide and/or be positioned into engagement with a side surface of second section 104b. More particularly, by retracting cable 106a, first section 104c is positioned against second section 104b such that the two pairs of opposing mechanical interfaces, namely, interface 124 of second section 104b and interface 126 of first section 104c, engage one another to position first section 104c in angular, preferably orthogonal orientation with respect to second section 104b. Cable 106a may be actuated after placing the distal end 101 adjacent an organ or tissue to be retracted so that the cable moves first section 104c and the organ or tissue simultaneously. Alternatively, the retractor may be moved into the second configuration and then engaged with an organ or tissue to be retracted. Then retractor 100 is moved to move the organ or tissue.

As can be appreciated, while the particular configurations of first and second sections 104c and 104b are shown at a substantially 90° angle relative to one another, it is envisioned and within the scope of the present disclosure that the orientation of first section 104c relative to second sections 104b can be at any angle "a" relative to longitudinal axis "A" (See FIG. 1B). Cable 106a may be subsequently locked to securely affix second and first sections 104b and 104c relative to one another for retraction purposes.

Once first section 104c has been engaged with intermediate section 104b, the surgeon remotely actuates second cable 106b (e.g., pulls on second cable 106b in a proximal direction) thereby causing second section 104b to translate and/or be positioned into engagement with proximal section 104a along longitudinal axis "A". In particular, as best seen in FIG. 1C, by retracting cable 106b, second section 104b is positioned against third section 104a such that the two pairs of opposing mechanical interfaces, namely, pair 120 of third section 104a and pair 122 of second section 104b, engage one another to align and secure second section 104b against third section 104a as best seen in FIG. 1C. Cable 106b may be subsequently locked to securely affix second section 104b relative to third section 104a for retraction purposes. First section 104c, second section 104b and third section 104a may be disengaged by releasing cable 106a and 106b so that the sections return and/or are free to return to the initial configuration under the action of gravity. Retractor 100 can then be removed from trocar assembly 10.

As can be appreciated, first and second cables 106a, 106b may also be remotely actuated in reverse order, i.e., 106b actuated first followed by first cable 106a, or simultaneously depending upon a particular purpose. Interfaces 120, 122, 124 and 126 may include any combination of one or more detents, flanges, pins, tabs, grooves, slots, or the like which complement one another and which securely engage third, second and first sections 104a, 104b and 104c with one another for retraction purposes.

Preferably, flexible tube 102 is biased in a generally linear and/or straight orientation along longitudinal axis "A" and is made from a material which easily flexes as needed during configuration of retractor 100 but returns to its original generally linear and/or straight orientation (see FIG. 1A) upon release of cables 106a and 106b. While tube 102 provides flexibility, when retractor 100 is in the second and/or assembled configuration, third, second and first sections 104a, 104b and 104c engage one another and provide retractor 100 with a degree of rigidity. In further embodiments, tube 102 may be omitted.

In a further embodiment of a retractor, a first section having a mechanical interface at a proximal end is disposed at a distal end of the retractor. A second section is disposed proximal to the first section. The second section has a second mechanical interface for engaging the first mechanical interface on the first section. A cable extends through the first and second sections and is offset from the longitudinal axis of the first and second sections. The second mechanical interface is disposed on a side surface of the second section and the cable is arranged so that upon pulling the cable in a proximal direction, the first mechanical interface is engaged with the second mechanical interface and the first section is disposed at an angle with a longitudinal axis of the retractor..

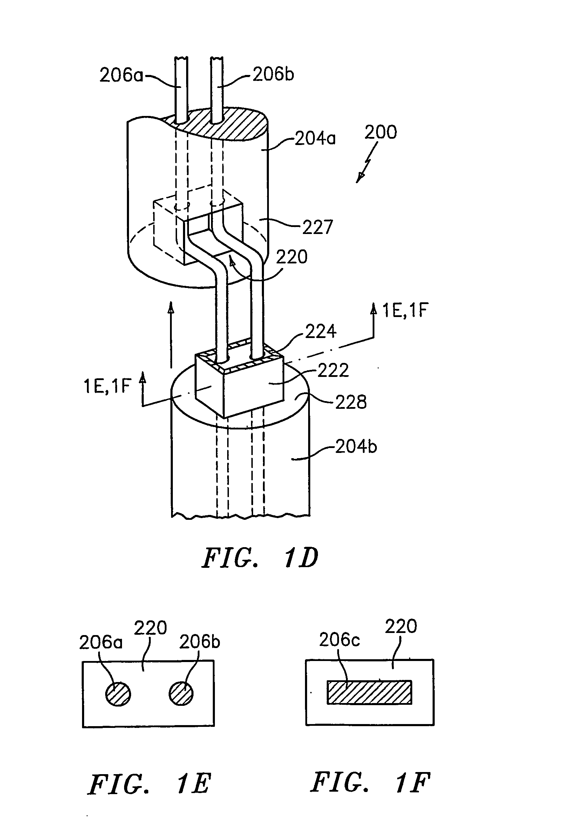

In a further embodiment shown in FIGS. 1D and 1E, a retractor 200 has a first section 204a and a second section 204b. As seen in FIG. 1D, first mechanical interface 220 is a socket for receiving second mechanical interface 222, which includes a tongue-like fitting disposed on and/or extending from a proximal surface 228 of second section 204b. Mechanical interface 220 is milled or otherwise formed, in a side surface 227 of first section 204a. It is envisioned that second mechanical interface 222 includes a chamfered edge 224 about the top periphery thereof which facilitates engagement with first mechanical interface 220 during assembly of sections 204a and 204b. One or more cables extend through both second mechanical interface 222 and first mechanical interface 220. Preferably, two cables 206a and 206b are provided for stability. Accordingly, when the surgeon actuates (e.g., pulls on) at least one of cables 206a, 206b, second section 204b is pulled towards first section 204a and second mechanical interface 222 inter-engages with first mechanical interface 220 to secure first and second sections 204a and 204b to one another for retraction purposes. Two or more such sections may be provided. It is envisioned that retractor 200 includes a flexible tube, like that shown in FIGS. 1A-1C. However, the flexible tube may be omitted.

In an alternate embodiment, as seen in cross-section in FIG. 1F, cables 206a, 206b are replaced with at least one ribbon-like cable 206c extending through each of second mechanical interface 222 and first mechanical interface 220.

As can be appreciated, cables 206a, 206b and/or ribbon-like cable 206c effectuates translation of first section 204a and second section 204b relative to one another during the engagement and/or disengagement process for retractor 200. A third section (not shown) may be provided in further embodiments.

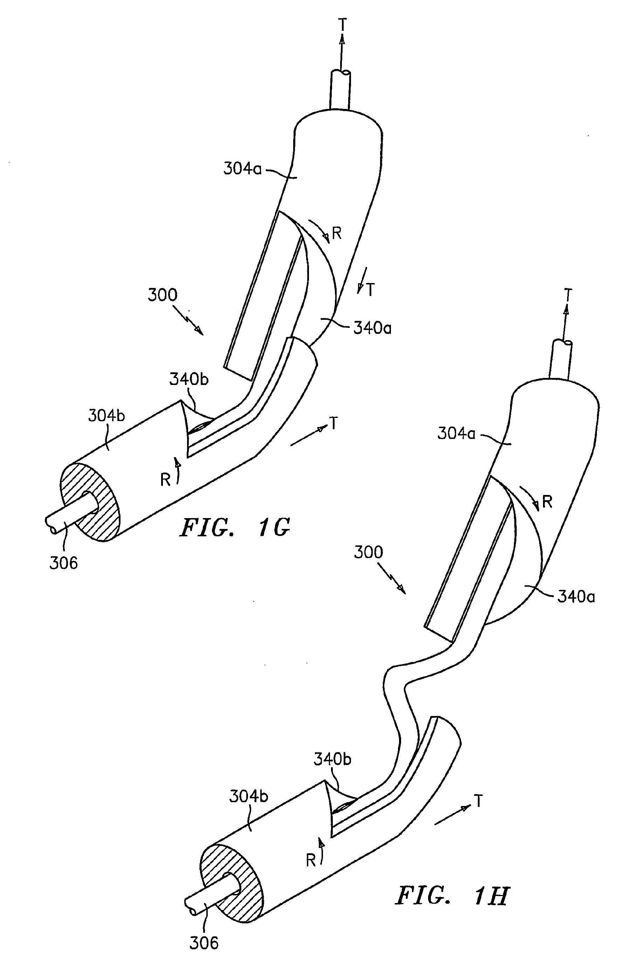

In a further embodiment shown in FIGS. 1G and 1H, a retractor 300 has a first section 304a and a second section 304b. In particular, first section 304a includes a first mechanical interface 320 including a helical cam-like surface 340a which engages a complementary cam-like surface 340b forming a second mechanical interface 322 for second section 304b. Accordingly, in use, surfaces 340a, 340b engage one another and rotate the first and second section 304a, 304b in the direction of arrows "R" upon translation of first section 304a and second section 304b towards one another in the direction of arrows "T".

A cable 306 is disposed along a central axis defined through each of first and second sections 304a and 304b and is utilized to effectuate translation and rotation of first section 304a and second section 304b relative to one another. As can be appreciated, surfaces 340a and 340b secure first section 304a and second section 304b in an interlocking, friction-fit manner for retraction purposes. Additionally or alternatively, cable 306 is secured to fix the relative positions of first section 304a and 304b. To disassemble retractor 300, as seen in FIG. 1H, the tension on the cable 306 is relaxed causing the sections to freely rotate and move away from one another in directions opposite to arrows "T" and "R".

Preferably, at least one of first and second sections 304a, 304b are arcuate in shape and/or are provided with a slight bend. In this manner, when second section 304b is mated with first section 304a, retractor 300 has a bent and/or arcuate configuration. Two or more such sections 304 may be provided. Desirably, retractor 300 includes a flexible tube like that shown in FIGS. 1A - 1C. However, the flexible tube may be omitted.

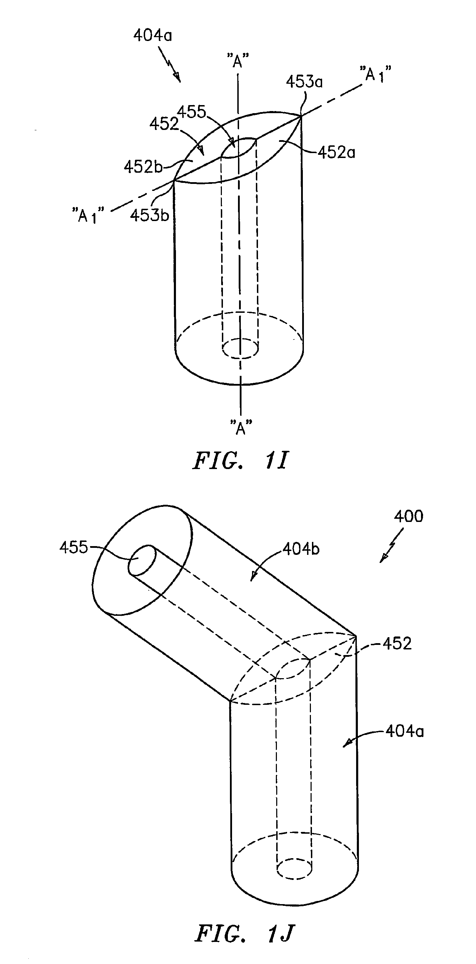

Turning now to FIG. 1I, an enlarged perspective view of first section 404a for a retractor 400, in accordance with another embodiment of the present disclosure, is shown. First section 404a includes a surface 452, desirably angled with respect to longitudinal axis "A". Angled surface 452 defines an upper tip 453a and a lower tip 453b. Preferably, angled surface 452 includes first and second surfaces 452a, 452b, each angled with respect to an axis "A₁" extending through upper and lower tips 453a, 453b.

Second section 404b preferably includes a surface (not shown) which complements angled surfaces 452a, 452b of first section 404a. Accordingly, in use, when a cable (not shown) extending through a passage 455 of first section 404a is actuated remotely by a surgeon, to approximate second section 404b to first section 404a (as seen in FIG. 1J), surfaces 452a, 452b inter-engage the complementary surfaces of second section 404b to secure sections 404a and 404b at an angle with respect to one another for retraction purposes. Two or more such sections 404 may be provided. Desirably, retractor 400 includes a flexible tube like that shown in FIGS. 1A - 1C. However, the flexible tube may be omitted.

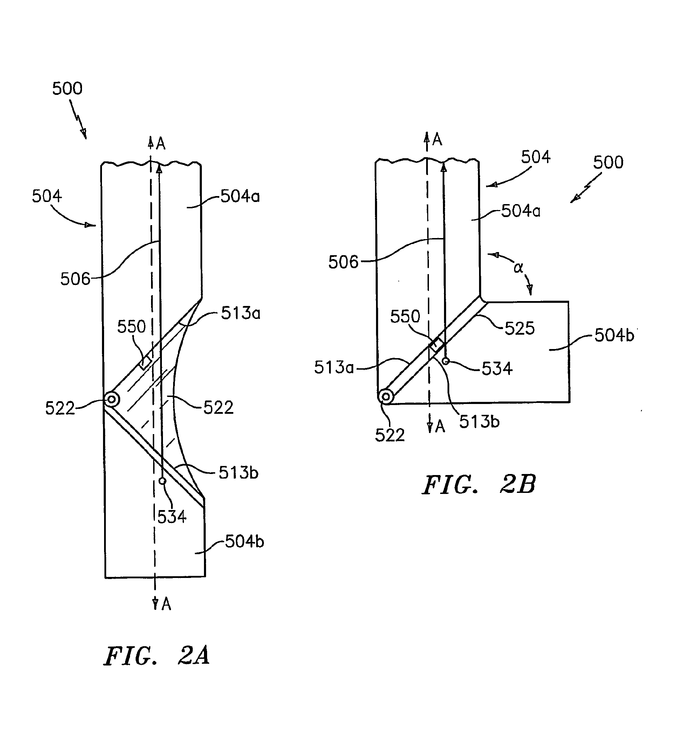

Turning now to FIGS. 2A and 2B, another embodiment of an endoscopic retractor in accordance with the present disclosure, is shown generally as 500. Organ retractor 500 includes an elongated shaft 504 having first and second sections 504a and 504b, respectively, pivotably connected to one another by a pivot member, preferably a hinge 522. Preferably, a film 525 extends between first and second sections 504a and 504b to reduce the susceptibility of organs and/or tissue from being pinched and/or caught between first and second sections 504a, 504b. Alternatively, first and second sections 504a, 504b may be covered by a flexible tube, as discussed above in connection with FIGS. 1A-1C. First and second sections 504a and 504b are rotatable relative to longitudinal axis "A" defined therethrough and about hinge 522 upon remote actuation of cable 506 by the surgeon. More particularly, as seen in FIG. 2B, cable 506 is affixed to second section 504b at point 534 such that selective translation (e.g., pulling) of cable 506 rotates second section 504b about pivot point 522. As can be appreciated, second section 504b may be rotated to various angles "a" relative to longitudinal axis "A" depending upon a particular purpose and depending on the particular dimensions and configuration of opposing surfaces 513a, 513b of first and second sections 504a and 504b, respectively. One or both of first and second sections 504a, 504b, respectively, may include a stop member 550 for limiting the degree of angular rotation "a" of second section 504b relative to first section 504a depending upon the particular purpose or to achieve a desired result.

As seen in FIG. 2C, shaft 504 of organ retractor 500 can include three sections, namely, proximal section 504a, intermediate section 504b, and distal section 504c. Proximal and intermediate sections 504a and 504b are pivotably connected about hinge 525 while intermediate and distal sections 504b and 504c are pivotably connected about a hinge 535. A first cable 506a is affixed to intermediate section 504b at point 534a and a second cable 506b is affixed to distal section 504c at a point 534b. Much like the aforementioned embodiments, cables 506a and 506b allow the surgeon to remotely assemble retractor 500 for manipulation of organs. More particularly, actuation of cable 506b rotates distal section 504c about pivot 535 such that a proximal surface thereof contacts and/or otherwise engages a distal surface of intermediate section 504b. Actuation of cable 506a rotates intermediate section 504b about pivot 525 such that a proximal surface thereof contacts and/or otherwise engages a distal surface of proximal section 504a.

It is envisioned that one or more sections 504a, 504b, or 504c may include a series of mechanical interfaces 540a, 540b, 542a and 542b which facilitate engagement and alignment of sections 504a, 504b, or 504c during configuration and/or assembly of retractor 500. For example, intermediate section 504b may include detents 540a and 540b which engage a complementary socket 542b disposed in distal section 504c and a complementary socket 542a disposed in proximal section 514a, respectively, upon formation of retractor 500. Alternatively, as seen in FIG. 2D, a tongue 550 may be utilized between proximal and intermediate sections 504a and 504b to assure proper and consistent rotation of intermediate section 504b relative to proximal section 504a during formation and/or configuration of retractor 500.

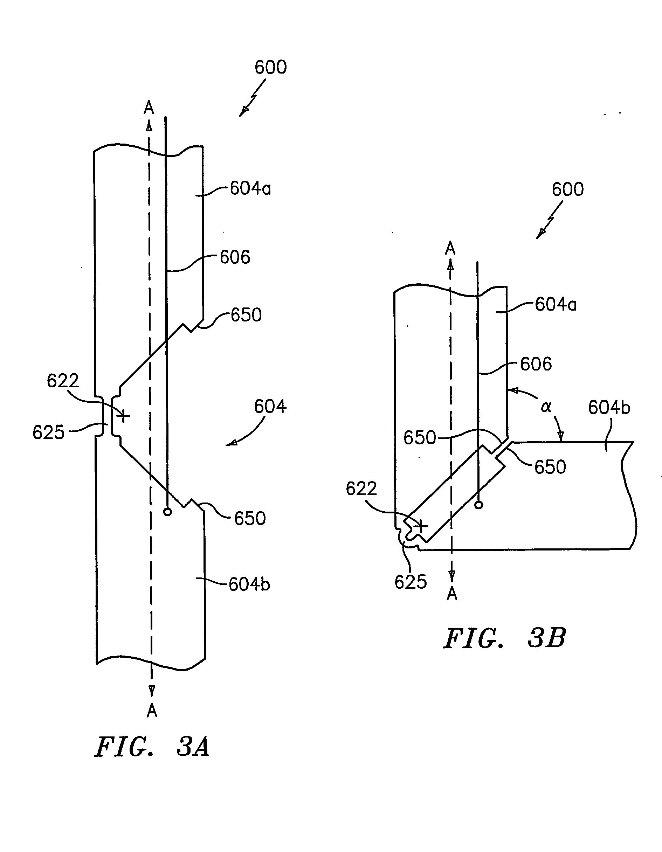

Turning now to FIGS. 3A and 3B, an endoscopic organ retractor, in accordance with yet another embodiment of the present disclosure, is designated generally as 600. Retractor 600 includes an elongated shaft 604 having first and second sections 604a and 604b, respectively, interconnected by a "living hinge" 625. A "living hinge" is a relatively thin portion of plastic or the like that bridges two relatively heavier and/or thicker walls and that provides the ability to repeatedly flex without the use of a mechanical hinge. First and second sections 604a, 604b are rotatable relative to a longitudinal axis "A" defined therethrough and about an imaginary pivot point 622 upon remote actuation of cable 606 by the surgeon. In particular, as seen in FIG. 3B, cable 606 is affixed to second section 614b at point 634 such that selective translation (e.g., pulling) of cable 606 biases second section 604b against living hinge 625 during rotation of second section 604b about imaginary pivot point 622. As can be appreciate, second section 604b may be rotated to various angles "a" relative to longitudinal axis "A" depending upon a particular purpose. One or both of first and second sections 604a, 604b, respectively, may include a stop member 650 for limiting the degree of angular rotation "a" of second section 604b depending upon a particular purpose or to achieve a desired result.

In the embodiments of FIGS. 2A-3B, the retractor may include a film extending between the sections, a flexible tube enclosing the sections of the retractor, or these feature may be omitted. In each of these embodiments, two or more sections may be provided in the retractor.

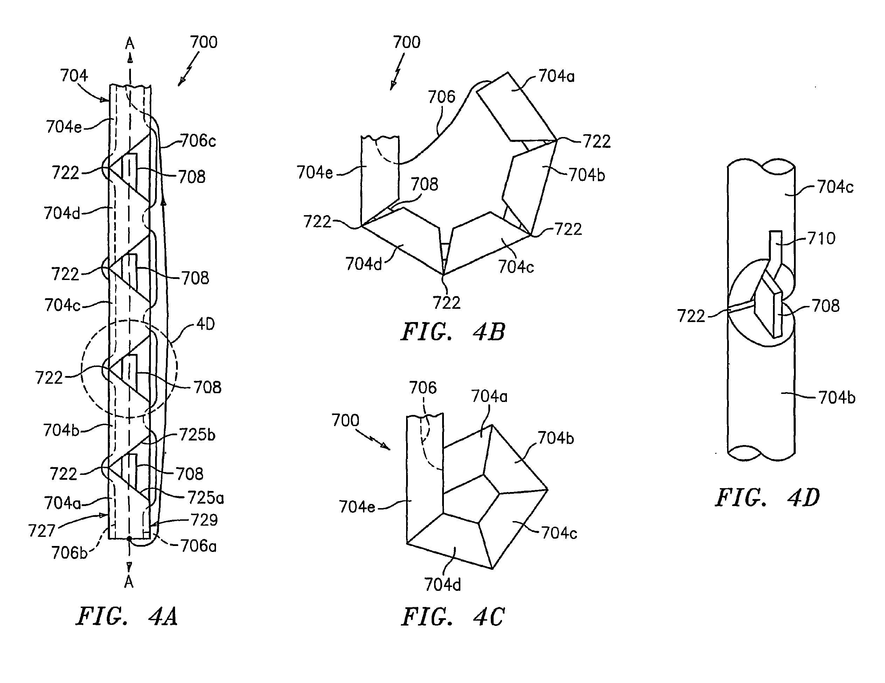

Turning now to FIGS. 4A - 4D, another embodiment of an endoscopic retractor in accordance with the present disclosure, is shown generally as 700. Retractor 700 includes an elongated shaft 704 having a plurality of sections 704a-704e pivotably connected to one another by a pivot member 722, preferably a hinge (e.g., a mechanical hinge, a living hinge, etc.). Retractor 700 has a first section 704a, a second section 704b, a third section 704c, a fourth section 704d and a fifth section 704e. However, people of ordinary skill in the field will appreciate that fewer or more sections may be used. Retractor 700 includes a cable 706 extending through the side of fifth section 704e, along the exterior of sections 704a-704d, and secured to the exterior surface of first section 704a. Each of sections 704 have a proximal end 725 and a distal end 728 that are angled, as opposed to obliquely oriented, with respect to longitudinal axis "A". As best seen in FIG. 4A, each section 704 has an angled distal end 728 that diverges from an angled proximal end 725 of an adjacent section. For example, distal end 728b (of section 704b) diverges from proximal end 725a (of section 704a), from a first side 727 to a second side 729 of retractor 700. The angled surfaces allow each section to rotate with respect to an adjacent section.

Sections 704a-704e are rotatable relative to longitudinal axis "A" defined therethrough and about pivot members 722 upon remote actuation of cable 706a by the surgeon. In particular, as cable 706a is drawn in a proximal direction, first section 704a is pulled towards and around to fifth section 704e, as seen in FIG. 4B. Preferably, cable 706c is drawn in a proximal direction until first section 704a contacts and/or rests against fifth section 704e. It is envisioned that the opposing surfaces of sections 704a-704e are angled an amount sufficient to enable first section 704a to contact fifth section 704e when retractor 700 is in a fully retracted condition.

As best seen in FIGS. 4A and 4D, each of sections 704a-704d of retractor 700 preferably include a tongue 708 extending therefrom in a direction for cooperative engagement with a complementary slot 710 formed in the adjacent section 704b-704e. For example, section 704b, has a tongue 708 at its proximal end, for engaging a slot 710 in the distal end of the third section 704c. Accordingly, when retractor 700 is in the fully retracted condition, as seen in FIG., 4C, tongues 708, in cooperation with slots 710, provide retractor 700 with increased rigidity and reduced susceptibility to twisting.

Retractor 700 further includes cable 706a extending through each section 704 and disposed between longitudinal axis "A" and second side 729 of the retractor 700. Cable 706b extends through each section 704 and is disposed between longitudinal axis "A" and first side 727. Preferably, cables 706a and 706b exit each section at a distal end of the section and enter the adjacent section at a proximal end of the section, as best seen in FIG. 4A. To bring retractor 700 from the first configuration (see FIG. 4A) to the second configuration (see FIG. 4C), cable 706a is pulled proximally, turning sections 704 about pivot members 722. To return retractor 700 to the first configuration, cable 706b is pulled proximally. In further embodiments, cable 706c may be provided without cables 706a and 706b. In other embodiments, cables 706a and 706b are provided without cables 706c. Desirably, retractor 700 includes a flexible tube like that shown in FIGS. 1A - 1C, however, the flexible tube may be omitted.

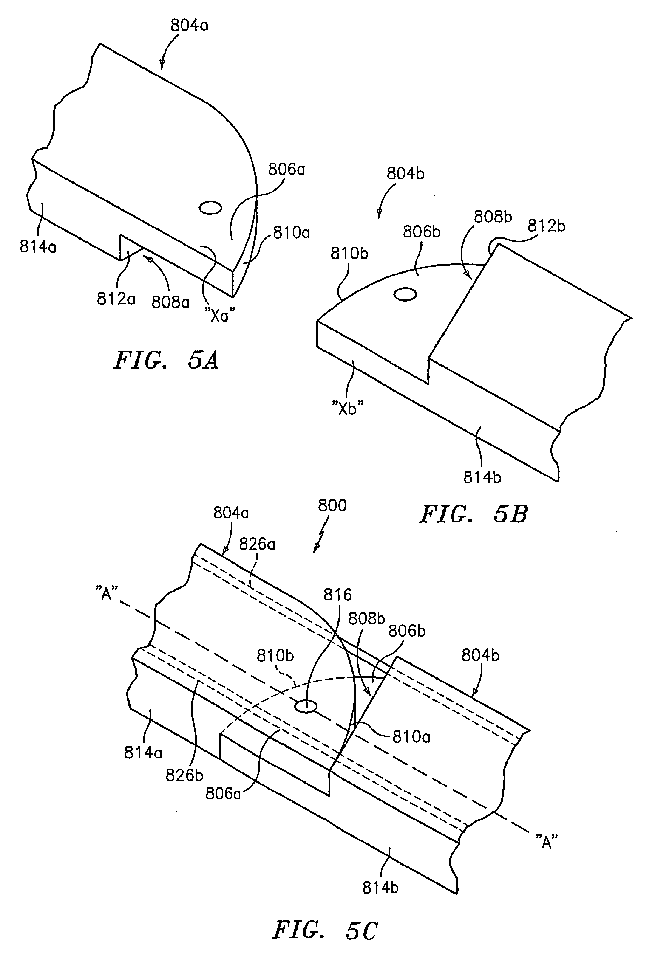

Turning now to FIGS. 5A - 5C, a segment of an endoscopic organ retractor 800, in accordance with another embodiment of the present disclosure, is shown. Retractor 800 includes at least a first section 804a and a second section 804b pivotably coupled to one another. As seen in FIG. 5A, first section 804a includes a tab, tongue or the like 806a extending longitudinally from a distal end 812a thereof. Tongue 806a defines a recess and/or cut-out 808a at the distal end of first section 804a. Tongue 806a includes an arcuate distal edge 810a. Arcuate distal edge 810a has a radius whose center "Xₐ" is desirably located at the intersection of a first side edge 814a of first section 804a and distal end 812a. As seen in FIG. 5B, second section 804b includes a tab, tongue or the like 806b extending longitudinally from a distal end 812b thereof. Tongue 806b defines a recess and/or cut-out 808b at the distal end of second section 804b. Tongue 806b includes an arcuate distal edge 810b. Arcuate distal edge 810b has a radius whose center "X_{b}" is desirably located at the intersection of a first side edge 814b of second section 804b (first side edge 814b being substantially aligned with first side edge 814a when first and second sections 804a, 804b are coupled together) and distal end 812b.

As seen in FIG. 5C, first and second sections 804a, 804b are pivotably coupled together by a pivot member 816 (e.g., a pivot pin) extending through tongues 806a, 806b. Preferably, when first and second sections 804a, 804b are coupled together, first side edge 814a is substantially aligned with first side edge 814b. In addition, arcuate distal edges 810a, 810b of tongues 806a, 806b, preferably over lap one another such that tongue 806a is disposed in recess 808b and tongue 806b is disposed in recess 808a. As best seen in FIG. 5C, desirably, distal edge 810a of tongue 806a contacts or lies adjacent to distal end 812b of second section 804b and distal edge 810b of tongue 806b contacts or lies adjacent to distal end 812a of first section 804a.

In this manner, retractor 800 is pivotable about pivot member 816 from a first position in which first section 804a is substantially longitudinally aligned with second section 804b, and any number of second positions, in which first section 804a is angled with respect to second section 804b. Tongues 806a, 806b inter-engage distal ends 812b, 812a in such a manner that first and second sections 804a, 804b pivot about pivot member 816 in a direction away from first side edges 814a, 814b. It is envisioned that retractor 800 can include a cable 826a extending through first section 804a and operatively connected to second section 804b. Cable 826a is offset from longitudinal axis "A" in such a manner so as to impart movement (i.e., pivoting) of second section 804b relative to first section 804a upon a pulling of cable 826a in a proximal direction, moving retractor 800 to the second configuration. Pulling of second cable 826b, which is disposed on an opposite side of axis "A" from cable 826a, returns retractor 800 to the first configuration. Alternatively, the second cable 826b may be omitted and the retractor may be returned to the first configuration by releasing first cable 826a and allowing the sections to move under the force of gravity. Two or more of such sections 804 may be provided to form an L-shaped retractor, as shown in FIG. 1C or a loop, as shown in FIG. 4C. A flexible tube, like that shown in FIGS. 1A - 1C, is desirably included in retractor 800. However, the flexible tube, like that shown in FIGS. 1A - 1C, may be omitted.

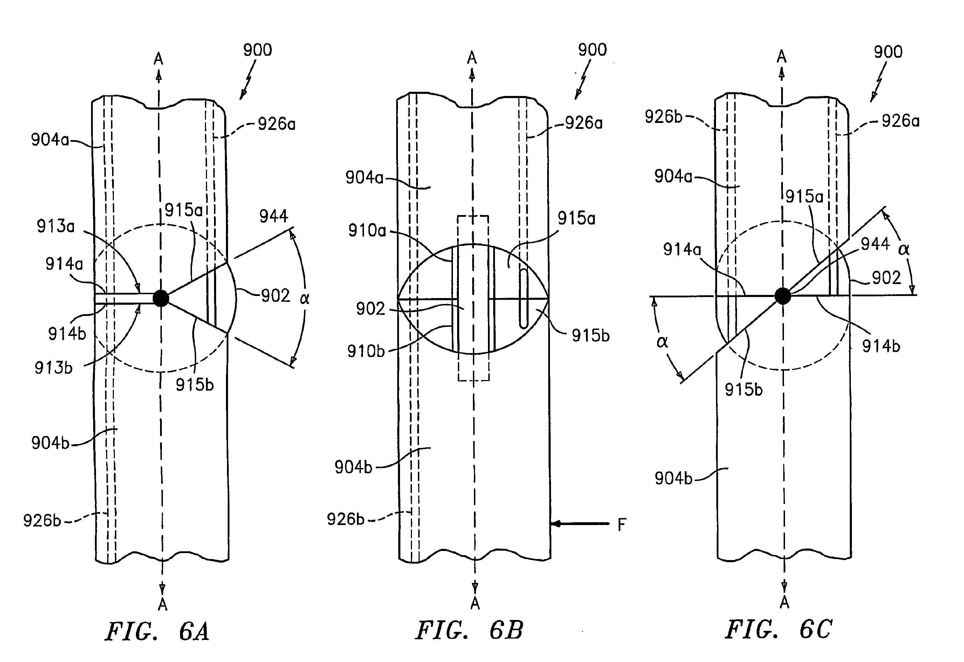

Turning now to FIGS. 6A - 6C, a segment of an endoscopic retractor 900, in accordance with another embodiment of the present disclosure, is shown. Retractor 900 includes at least a first section 904a and a second section 904b pivotably coupled to one another by a pivot member 944. As seen in FIGS. 6A and 6B, retractor 900 further includes a disc, wheel or the like 902 operatively disposed between first and second sections 904a, 904b so that the first section 904a is pivotably coupled with second section 904b.

First section 904a includes a distal surface 913a having a first surface 914a which is orthogonally oriented with respect to longitudinal axis "A" and a second surface 915a which is angled with respect to longitudinal axis "A". Second section 904b includes a proximal surface 913b having a first surface 914b which is orthogonally oriented with respect to longitudinal axis "A" and a second surface 915b which is angled with respect to longitudinal axis "A". Preferably, the central axis of disc 902 is orthogonally oriented with respect to longitudinal axis "A" and is positioned substantially at the intersection of first surfaces 914a, 914b and second surfaces 915a, 915b. Disc 902 is positioned within recesses 910a and 910b formed in first and second sections 904a, 904b, respectively. Disc 902 provides retractor 900 with a degree of rigidity when acted on by forces acting in a direction substantially parallel to the central axis of disc 902, as indicated by arrow F in FIG. 6B.

In one embodiment, as seen in FIGS. 6A and 6B, first surface 914a of first section 904a is juxtaposed relative to first surface 914b of second section 904b and second surface 915a of first section 904a is juxtaposed relative to second surface 915b of second section 904b. In this manner, retractor 900 is pivotable about the central axis of disc 902 from a first position in which first and second sections 904a, 904b are substantially aligned with one another and any number of second positions in which first and second sections 904a, 904b are pivoted about the central axis of disc 902 in order to be angled with respect to one another. First surfaces 914a, 914b engage one another and prevent first and second sections 904a, 904b from pivoting in a direction towards first and second surfaces 914a, 914b. Moreover, the angle of second surfaces 915a, 915b determines the angle and/or degree "a" to which retractor 900 can be bent.

Alternatively, as seen in FIG. 6C, first surface 914a of first section 904a is juxtaposed relative to second surface 915b of second section 904b and second surface 915a of first section 904a is juxtaposed relative to first surface 914b of second section 904b. In this manner, retractor 900 is pivotable about the central axis of disc 902 from a first position in which first and second sections 904a, 904b are substantially aligned with one another and any number of second positions in which first and second sections 904a, 904b are pivoted about the central axis of disc 902 in order to be angled with respect to one another. The position of second surfaces 915a, 915b of first and second sections 904a, 904b enables retractor 900 to be bent by an angle and/or degree "a" to either side thereof (i.e., in the direction of first surface 914a or in the direction of second surface 915a).

It is envisioned that retractor 900 includes a cable 926a extending through first section 904a and operatively connected to second section 904b. Cable 926a is offset from longitudinal axis a in such a manner so as to impart movement (i.e., pivoting) of second section 904b relative to first section 904a upon a pulling of cable 926 in a proximal direction, moving retractor 900 to the second configuration. A second cable 926b is offset from longitudinal axis "A" in a second direction from cable 926a, so that pulling cable 926b returns retractor 900 to the first configuration. Alternatively, the second cable 926b may be omitted and the retractor may be returned to the first configuration by releasing the first cable 926a and allowing the sections to move under the force of gravity. Retractor 900 may include two or more of the sections 904, to provide an L-shaped retractor as shown in FIG. 1C, or a loop-shaped retractor as shown in FIG. 4C. Retractor 900 may also include a flexible tube, like that shown in FIGS. 1A - 1C. However, the flexible tube may be omitted.

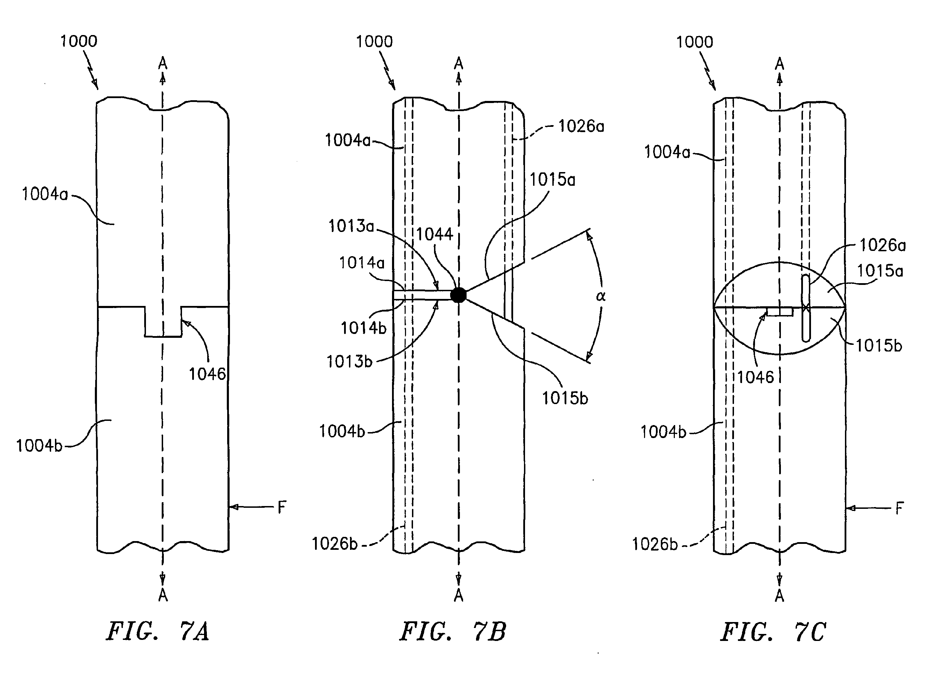

Turning now to FIGS. 7A - 7C, a segment of an endoscopic retractor 1000, in accordance with another embodiment of the present disclosure, is shown. Retractor 1000 includes at least a first section 1004a and a second section 1004b pivotably coupled to one another by a pivot member 1044. As seen in FIG. 7A, first and second sections 1004a, 1004b of retractor 1000 are joined together by a knuckle joint 1046 (e.g., tongue and groove, dove-tail, etc.).

First section 1004a includes a distal surface 1013a having a first surface 1014a which is orthogonally oriented with respect to longitudinal axis "A" and a second surface 1015a which is angled with respect to longitudinal axis "A". Second section 1004b includes a proximal surface 1013b having a first surface 1014b which is orthogonally oriented with respect to longitudinal axis "A" and a second surface 1015b which is angled with respect to longitudinal axis "A". The angle of second surfaces 1015a, 1015b determines the angle and/or degree "a" to which retractor 1000 can be bent. Joint 1046 provides retractor 1000 with a degree of rigidity when acted on by forces acting in a direction substantially parallel to the axis of rotation of first and second sections 1004a, 1004b, as indicated by arrow F in FIGS. 7A and 7C.

It is envisioned that retractor 1000 includes a cable 1026a extending through first section 1004a and operatively connected to second section 1004b. Cable 1026a is offset from longitudinal axis "A" in such a manner so as to impart movement (i.e., pivoting) of second section 1004b relative to first section 1004a upon a pulling of cable 1026a in a proximal direction, moving retractor 1000 to the second configuration. A second cable 1026b is offset from axis "A" in an opposite direction from cable 1026a, so that pulling on cable 1026b returns retractor 1000 to the first configuration. Alternatively, the second cable 1026b may be omitted and the retractor is returned to the first configuration by releasing the first cable 1026a and allowing the sections to move under the force of gravity. Retractor 1000 may include two or more sections 1004, to provide an L-shaped retractor as shown in FIG. 1C, or a loop-shaped retractor, as shown in FIG. 4C. Desirably, retractor 1000 includes a flexible tube like that shown in FIGS. 1A - 1C. However, the flexible tube may be omitted.

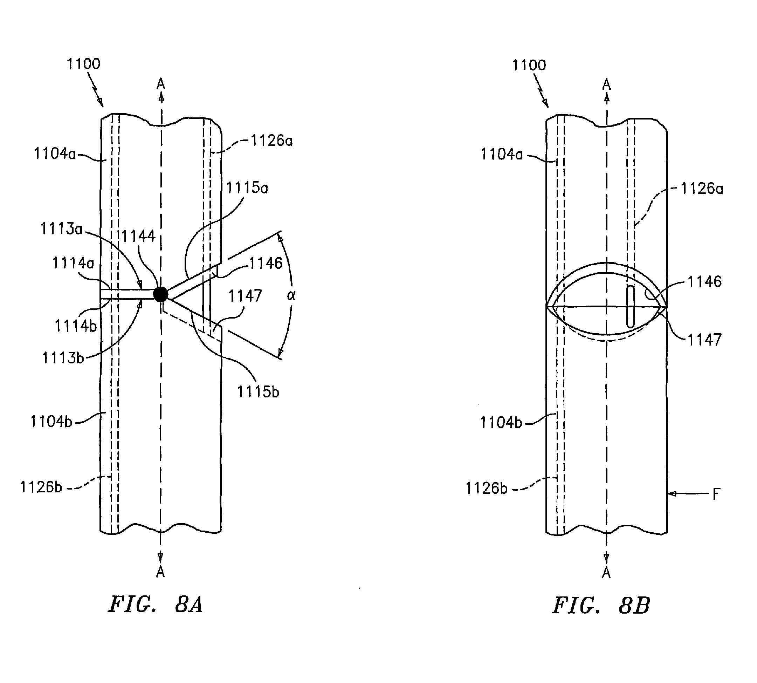

Turning now to FIGS. 8A and 8B, a segment of an endoscopic retractor 1100, in accordance with another embodiment of the present disclosure, is shown. Retractor 1100 includes at least a first section 1104a and a second section 1104b pivotably coupled to one another by a pivot member 1144. First section 1104a includes a distal surface 1113a having a first surface 1114a which is orthogonally oriented with respect to longitudinal axis "A" and a second surface 1115a which is angled with respect to longitudinal axis "A". Second section 1104b includes a proximal surface 1113b having a first surface 1114b which is orthogonally oriented with respect to longitudinal axis "A" and a second surface 1115b which is angled with respect to longitudinal axis "A". The angle of second surfaces 1115a, 1115b determines the angle and/or degree "a" to which retractor 1100 can be bent.

Retractor 1100 further includes a tab 1146 extending from one of second surface 1115a of first section 1104a or second surface 1115b of second section 1104b. Retractor 1100 further includes a recess or depression 1147 formed in the other of second surface 1115a of first section 1104a and second surface 1115b of second section 1104b. Preferably, tab 1146 is complementary in shape to recess 1147. Tab 1146 and recess 1147 provide retractor 1100 with a degree of rigidity, when in the bent configuration, when acted on by forces acting in a direction substantially parallel to the axis of rotation of first and second sections 1104a, 1104b, as seen in FIG. 8B.

It is envisioned that retractor 1100 includes a cable 1126a extending through first section 804a and operatively connected to second section 1104b. Cable 1126a is offset from longitudinal axis "A" in such a manner so as to impart movement (i.e., pivoting) of second section 1104b relative to first section 1104a upon a pulling of cable 1126 in a proximal direction to move retractor 1100 to the second configuration. Retractor 1100 has a second cable 1126b offset from axis "A" in an opposite direction from cable 1126a so that pulling on cable 1126b returns retractor 1100 to the first configuration. Alternatively, the second cable 1126b may be omitted and the retractor is returned to the first configuration by releasing the first cable 1126a and allowing the sections to move under the force of gravity. Retractor 1100 may include two or more sections 1104 to provide an L-shaped retractor, as shown in FIG. 1C, or a loop-shaped retractor, as shown in FIG. 4C. Desirably, retractor 1100 includes a flexible tube, as shown in FIGS. 1A - 1C. However, the flexible tube may be omitted.

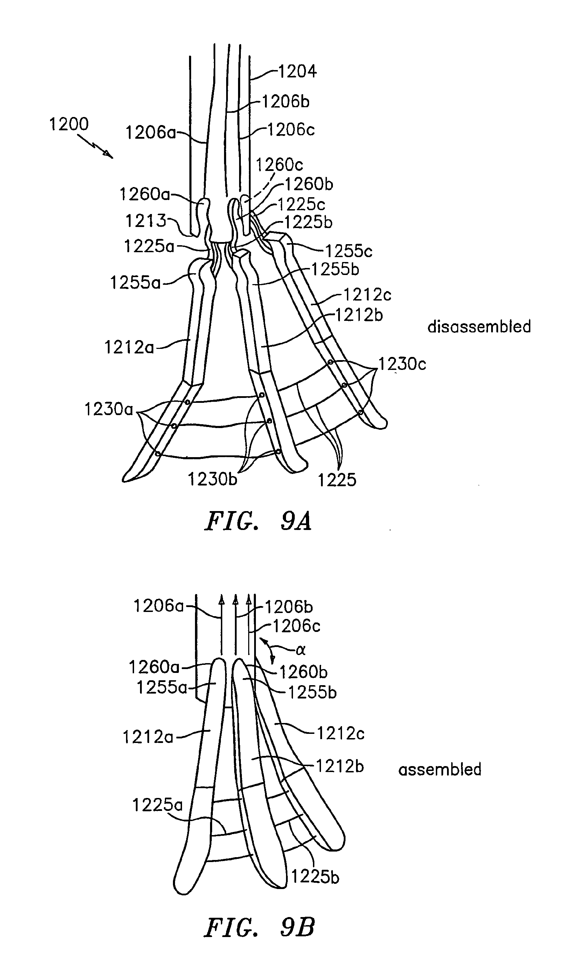

Turning now to FIGS. 9A and 9B, an endoscopic retractor, in accordance with yet another embodiment of the present disclosure, is generally designated as retractor 1200. Retractor 1200 includes an elongated shaft 1204, and a plurality of finger elements 1212a, 1212b and 1212c which extend from and are operatively engagable with a distal end 1213 of shaft 1204. Retractor 1200 also includes a plurality of cables 1206a, 1206b and 1206c disposed therethrough which are remotely operable by the surgeon to form, assemble and/or configure retractor 1200 after insertion through a trocar assembly (not shown). Each cable 1206a-1206c includes a bundle of cords 1225a-1225c which extend from a respective cable 1206a-1206c and into a corresponding finger element 1212a-1212c. Each bundle of cords 1225a-1225c, in turn, separates into individual cord elements (not shown) which ultimately connect to and/or inter-connect adjacent finger elements 1212a-1212c to one another through a series of side ports 1230a-1230c formed in each finger element 1212a-1212c, respectively.

As best illustrated in FIG. 9B, after the surgeon inserts retractor 1200 through the trocar assembly (not shown), the surgeon simply pulls cables 1206a, 1206b and 1206c in a proximal direction to form the supporting structure of retractor 1200. In particular, by pulling cables 1206a-1206c in a proximal direction, the corresponding bundle of cords 12925a-1225c are also pulled proximally which, in turn, pull finger elements 1212a-1212c into engagement with distal end 1213 of shaft 1204 and pull the adjacent finger elements 1212a-1212c, into tight cooperation with one another to facilitate organ retraction. As can be appreciated, cables 1206a-1206c can be actuated simultaneously or sequentially depending upon a particular purpose.

As best seen in FIG. 9A, it is envisioned that distal end 1213 of shaft 1214 may include a series of key-like sockets 1260a, 1260b and 1260c which mate with a corresponding flanges 1255a, 1255b and 1255c formed at a proximal end of each finger element 1212a-1212c, respectively. Each flange 1255a-1255c may be shaped to interface with a corresponding socket 1260a-1260c such that the corresponding finger element 1212a-1212c, when engaged with distal end 1213 of shaft 1214, is disposed at a particular angle "a" relative to a longitudinal axis of shaft 1214 in order to facilitate retraction and handling of a body organ.

Each finger element 1212a-1212c may comprise a plurality of sections having inter-engaging interfaces and a plurality of cables (e.g., 1206a-1206c) for articulating the sections with respect to one another. The inter-engaging interfaces may comprise any pair of complementary shapes on adjacent sections. The sections may be connected by a living hinge or mechanical hinge, or may be unconnected, as discussed above. Desirably, finger elements 1212a-1212c articulate with respect to shaft 1204 by operation of cables 1206a-1206c.

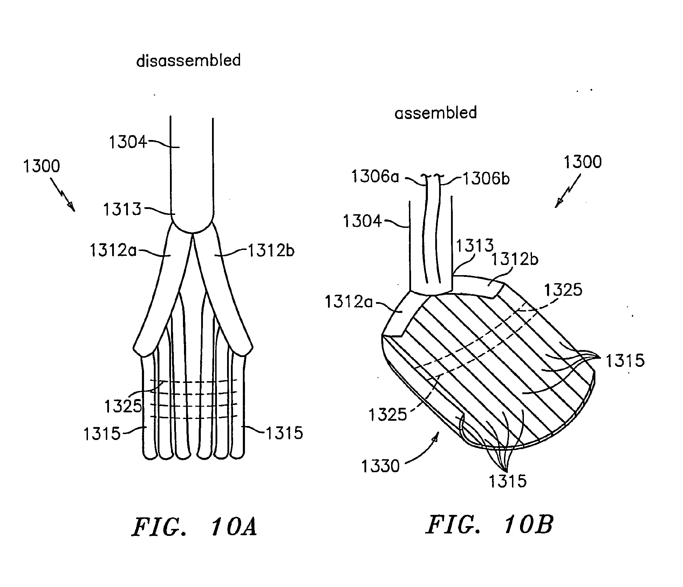

Turning now to FIGS. 10A and 10B, an endoscopic retractor, in accordance with yet another embodiment of the present disclosure, is generally designated 1300. Retractor 1300 includes a shaft 1304, and at least a pair of cables 1306a and 1306b disposed therethrough. Cables 1306a, 1306b are remotely operable by a surgeon to assemble and disassemble retractor 1300 as needed during surgery. Retractor 1000 further includes a pair of arms and/or plates 1312a and 1312b pivotably affixed to a distal end 1313 of shaft 1314. Plates 1312a, 1312b are movable from a first orientation (i.e., having a reduced-diameter, as seen in Fig. 10A, in which finger elements 1315 are substantially aligned with the longitudinal axis, to facilitate insertion through a trocar assembly (not shown) to a second orientation (i.e., expanded), in which finger elements 1315 are at an angle with respect to the longitudinal axis, to facilitate retraction of tissue and organs during surgery (see Fig. 10B). As seen in FIG. 10B, each plate 1312a and 1312b includes a plurality of finger elements 1315 affixed thereto which, when plates 1312a and 1312b are expanded to the second configuration, mutually cooperate to form a scoop-like structure or trowel 1330, which enhances organ retraction. It is envisioned that the plurality of finger elements 1315 are connected to one another by a series of wires 1325 (or the like) which finger elements 1315 become rigid upon expansion of the plates 1312a and 1312b to provide additional support for scoop-like structure 1330.

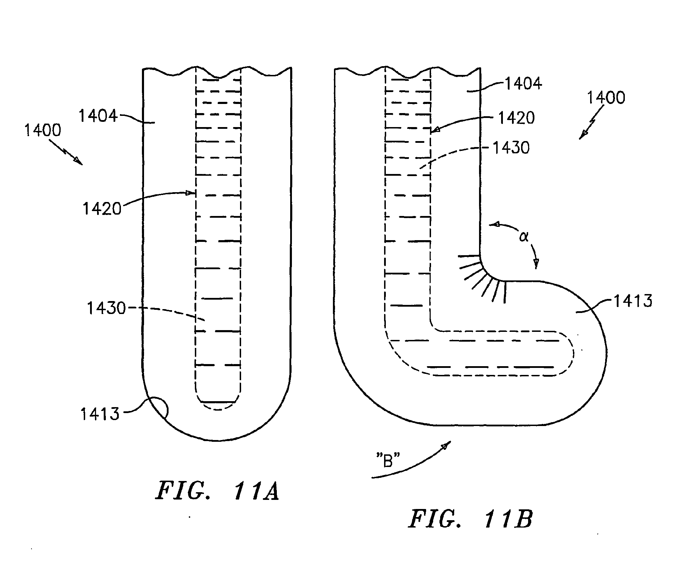

Turning now to FIGS. 11A and 11B, an endoscopic retractor, in accordance with an alternate embodiment, is generally designated as 1400. Retractor 1400 includes an elongated shaft 1404 having a bore, lumen and/or elongate recess 1420 formed therethrough and which contains a liquid 1430 retained in recess 1420 thereof. A distal end 1413 of shaft 1414 is preferably made from a shape memory alloy such that upon a change in temperature of liquid 1430 within recess 1420, distal end 1413 of shaft 1414 transforms and/or configures into a scoop-like configuration, as indicated by arrow "B", for retracting organs. It is envisioned that distal end 1413 can be configured to have any angle "a" corresponding to a specific purpose or to achieve a particular result.

Shape memory alloys (SMAs) transform in shape when changed from an austenitic state to a martenistic state due to a change in temperature. SMAs are a family of alloys having anthropomorphic qualities of memory and trainability and are particularly well suited for use with medical instruments. SMAs have been applied to such items as actuators for control systems, steerable catheters, and clamps. One of the most common SMAs is Nitinol which can retain shape memories for two different physical configurations and changes shape as a function of temperature.

Recently, other SMAs have been developed based on copper, zinc and aluminum and have similar shape memory retaining features. SMAs undergo a crystalline phase transition upon applied temperature and/or stress variations. A particularly useful attribute of SMAs is that after it is deformed by temperature/stress, it can be completely recover to its original shape upon its return to the original temperature. This transformation is referred to as a thermoelastic martenistic transformation.

Under normal conditions, the thermoelastic martenistic transformation occurs over a temperature range which varies with the composition of the alloy, itself, and the type of thermal-mechanical processing by which it was manufactured. In other words, the temperature at which a shape is "memorized" by an SMA is a function of the temperature at which the martensite and austenite crystals form in that particular alloy. For example, Nitinol alloys can be fabricated so that the shape memory effect will occur over a wide range of temperatures, e.g., about -270 to about +100 degrees Celsius.

It is further envisioned that the shape memory alloy can be replaced with a shape memory plastic when forming an endoscopic organ retractor for use in manipulating organs. Shape memory plastics are polymeric materials which exhibit the property of shape memory similar to that of shape memory alloys.

It will be understood that various modifications may be made to the various embodiments shown herein. In further embodiments of the invention, an instrument comprises an articulating shaft, including a plurality of sections having inter-engaging interfaces and a plurality of cables connected to the sections for articulating the sections with respect to one another. Therefore, the above description should not be construed as limiting, but merely as exemplifications of preferred embodiments. Those skilled in the art will envision other modifications within the scope of the claims appended hereto.

## Claims

1. A retractor (706) for use through a trocar port, comprising:
a shaft (704) having at least a first section (704a-e) having a first mechanical interface, a second section (704 a-e) having a second mechanical interface for engaging the first mechanical interface, and further comprising a hinge (722) disposed between the first section and the second section, the first section and second section being selectively rotatable about the hinge from a first, generally longitudinally-aligned configuration along an axis defined through the shaft and the first mechanical interface is disengaged from the second mechanical interface, to a second configuration wherein the second section is disposed at an angle relative to a longitudinal axis of the shaft and the first mechanical interface is engaged with the second mechanical interface; and
at least one first cable (706) extending through the shaft and being operatively secured to the second section, the first cable being remotely actuatable to move the second section from the first to the second configuration upon selective translation of the first cable, **characterized in that**
at least one of the first section and the second section include a tongue (708) which engages a corresponding recess (710) disposed within the other of the first section and the second section to facilitate alignment and engagement of the first section and the second section relative to one another during rotation from the first configuration to the at least one additional second configuration and to provide rigidity when acted on by forces acting in a direction substantially parallel to an axis of rotation of the first and second sections.

2. A retractor according to claim 1 wherein the first and second mechanical interfaces cooperate to align the first section and the second section and engage the first and second sections with one another upon movement from the first configuration to the second configuration.

3. A retractor according to claim 1 wherein the hinge is a living hinge (722) disposed between the first section and the second section.

4. The retractor of claim 1, further comprising: a third section (504a-e) adjacent the second section, the third section having a third mechanical interface for engaging a fourth mechanical interface of said second section; and a second cable (506a, 506b) operatively secured to the third section, wherein translation of the second cable causes the third section to operatively engage the second section.

5. The retractor of claim 4, wherein the third mechanical interface is complementary with the fourth mechanical interface, wherein when the second and third sections engage one another, the third mechanical interface and the fourth mechanical interface maintain the third section at an angle with respect to the longitudinal axis.

## Patentansprüche

1. Retraktor (706) zur Verwendung durch einen Trokaranschluss, umfassend:
einen Schaft (704) mit zumindest einem ersten Abschnitt (704a-e) mit einer ersten mechanischen Zwischenfläche, einem zweiten Abschnitt (704a-e) mit einer zweiten mechanischen Zwischenfläche zum Ergreifen der ersten mechanischen Zwischenfläche und ferner umfassend ein Gelenk (722), das zwischen dem ersten Abschnitt und dem zweiten Abschnitt angeordnet ist, wobei der erste Abschnitt und der zweite Abschnitt wahlweise um das Gelenk drehbar ist aus einer ersten, allgemein in Längsrichtung fluchtenden Konfiguration entlang einer Achse, die durch den Schaft definiert ist, und in der die erste mechanische Zwischenfläche von der zweiten mechanischen Zwischenfläche entfernt ist, in eine zweite Konfiguration, wobei der zweite Abschnitt in einem Winkel relativ zu einer Längsachse des Schafts angeordnet ist und die erste mechanische Zwischenfläche mit der zweiten mechanischen Zwischenfläche in Eingriff ist; und
zumindest ein erstes Kabel (706), das sich durch den Schaft erstreckt und an dem zweiten Abschnitt wirkend angebracht ist, wobei das erste Kabel von Ferne betätigbar ist, um den zweiten Abschnitt nach einer wahlweisen Verschiebung des ersten Kabels aus der ersten in die zweite Konfiguration zu bewegen, **dadurch gekennzeichnet, dass**
zumindest einer des ersten Abschnitts und des zweiten Abschnitts eine Zunge (708) aufweist, die in eine entsprechende Ausnehmung (710) eingreift, die innerhalb des anderen des ersten Abschnitts und des zweiten Abschnitts angeordnet ist, um eine Flucht und ein Eingreifen des ersten Abschnitts und des zweiten Abschnitts relativ zueinander während einer Rotation aus der ersten Konfiguration in die zumindest eine zusätzliche zweite Konfiguration zu erleichtern und Steifigkeit zu liefern, wenn auf sie durch Kräfte eingewirkt wird, die in eine im Wesentlichen parallel zu der Rotationsachse des ersten und zweiten Abschnitts liegende Richtung wirken.

2. Retraktor nach Anspruch 1, wobei die erste und zweite mechanische Zwischenfläche zusammenwirken, um den ersten Abschnitt und den zweiten Abschnitt nach einer Bewegung aus der ersten Konfiguration in die zweite Konfiguration miteinander fluchtend zu bringen und den ersten und zweiten Abschnitt miteinander in Eingriff zu bringen.

3. Retraktor nach Anspruch 1, wobei das Gelenk ein Foliengelenk (722) ist, das zwischen dem ersten Abschnitt und dem zweiten Abschnitt angeordnet ist.

4. Retraktor nach Anspruch 1, ferner umfassend einen dritten Abschnitt (504a-e) neben dem zweiten Abschnitt, wobei der dritte Abschnitt eine dritte mechanische Zwischenfläche zum Ergreifen einer vierten mechanischen Zwischenfläche des zweiten Abschnitts aufweist; und ein zweites Kabel (506a, 506b), das an dem dritten Abschnitt wirkend befestigt ist, wobei eine Verschiebung des zweiten Kabels den dritten Abschnitt dazu bringt, den zweiten Abschnitt wirkend zu ergreifen.

5. Retraktor nach Anspruch 4, wobei die dritte mechanische Zwischenfläche komplementär zu der vierten mechanischen Zwischenfläche ist, wobei, wenn der zweite und dritte Abschnitt ineinander eingreifen, die dritte mechanische Zwischenfläche und die vierte mechanische Zwischenfläche den dritten Abschnitt in einem Winkel in Bezug auf die Längsachse aufrechterhalten.

## Revendications

1. Ecarteur (706) pour utilisation à travers un orifice de trocart, comprenant:
un arbre (704) ayant au moins une première section (704a-e) ayant une première interface mécanique, une deuxième section (704a-e) ayant une deuxième interface mécanique pour la mise en prise avec la première interface mécanique, et comprenant en outre une articulation (722) disposée entre la première section et la deuxième section, la première section et la deuxième section pouvant tourner sélectivement autour de l'articulation d'une première configuration alignée généralement longitudinalement le long d'un axe défini par l'arbre, et la première interface mécanique est sortie de prise avec la deuxième interface, à une seconde configuration dans laquelle la seconde section est disposée à un angle relativement à un axe longitudinal de l'arbre, et la première interface mécanique est en prise avec la seconde interface mécanique; et
au moins un premier câble (706) s'étendant à travers l'arbre et étant fonctionnellement fixé à la seconde section, le premier câble étant actionnable à distance pour amener la seconde section de la première à la seconde configuration lors d'une translation sélective du premier câble, **caractérisé en ce que**
au moins une parmi la première section et la seconde section comporte une languette (708) qui s'engage dans un évidement correspondant (710) disposé dans l'autre parmi la première section et la seconde section pour faciliter l'alignement et la mise en prise de la première section et de la seconde section l'une relativement à l'autre durant la rotation de la première configuration à la au moins une seconde configuration additionnelle et pour fournir de la rigidité lorsque des forces agissent sur celle-ci agissent dans une direction sensiblement parallèle à un axe de rotation des première et seconde sections.

2. Ecarteur selon la revendication 1, dans lequel les première et seconde interfaces mécaniques coopèrent pour aligner la première section et la seconde section et pour mettre en prise les première et seconde sections l'une avec l'autre lors d'un mouvement de la première configuration à la seconde configuration.

3. Ecarteur selon la revendication 1, dans lequel l'articulation est une articulation vive (722) disposée entre la première section et la seconde section.

4. Ecarteur selon la revendication 1, comprenant en outre: une troisième section (504a-e) adjacente à la deuxième section, la troisième section ayant une troisième interface mécanique pour la mise en prise avec une quatrième interface mécanique de ladite deuxième section; et un deuxième câble (506a, 506b) fonctionnellement fixé à la troisième section, où la translation du deuxième câble amène la troisième section à venir fonctionnellement en prise avec la deuxième section.

5. Ecarteur selon la revendication 4, dans lequel la troisième interface mécanique est complémentaire à la quatrième interface mécanique, ou lorsque les deuxième et troisième sections viennent en prise l'une avec l'autre, la troisième interface mécanique et la quatrième interface mécanique maintiennent la troisième section à un angle par rapport à l'axe longitudinal.
